# EUROPÄISCHE PATENTANMELDUNG

(11) **EP 2 360 230 A1**
(43) Veröffentlichungstag der Anmeldung: **24.08.2011**
(21) Anmeldenummer: 10153728.0
(22) Anmeldetag: 16.02.2010
(51) Int. Cl.: C10G 2/00, C07C 1/04, C07C 29/151

(54) **Verfahren und Vorrichtung zur Verwertung von Emissionen eines Kraftwerks**

(71) Anmelder: Siemens Aktiengesellschaft, 80333 München (DE)
(72) Erfinder: Waidhas, Manfred Dr., 90427 Nürnberg (DE); Wegener, Dieter Dr., 82166 Gräfelfing (DE)

(57) **Zusammenfassung**

Die Erfindung betrifft ein Verfahren und eine Vorrichtung zur Verwertung von Emissionen eines Kraftwerks, das mit mindestens einem Brennstoff auf Kohlenstoffbasis betrieben wird. Das Kraftwerk umfasst mindestens einen Emittenten, welcher Emissionen umfassend ein Kohlenstoffdioxid enthaltendes Abgas erzeugt, mindestens eine Elektrolyseeinrichtung zur Zerlegung von Wasser in Wasserstoff und Sauerstoff, und mindestens einen, dem mindestens einen Emittenten und der mindestens einen Elektrolyseeinrichtung nachgeschalteten chemischen Reaktor zur Umsetzung zumindest eines Teils des Abgases und des Wasserstoffs in mindestens einen synthetischen Grund- oder Brennstoff. Dabei wird der mindestens einen Elektrolyseeinrichtung und/oder dem mindestens einen chemischen Reaktor, zusätzlich zu einer ersten Emission umfassend mindestens den Teil des Abgases, mindestens eine zweite Emission des mindestens einen Emittenten zur Verwertung zugeführt.

## Beschreibung

Verfahren und Vorrichtung zur Verwertung von Emissionen eines Kraftwerks

Die Erfindung betrifft ein Verfahren zur Verwertung von Emissionen eines mit mindestens einem Brennstoff auf Kohlenstoffbasis betriebenen Kraftwerks, umfassend mindestens einen Emittenten, welcher Emissionen umfassend ein Kohlenstoffdioxid enthaltendes Abgas erzeugt, mindestens eine Elektrolyseeinrichtung zur Zerlegung von Wasser in Wasserstoff und Sauerstoff, und mindestens einen, dem mindestens einen Emittenten und der mindestens einen Elektrolyseeinrichtung nachgeschalteten chemischen Reaktor zur Umsetzung zumindest eines Teils des Abgases und des Wasserstoffs in mindestens einen synthetischen Grund- oder Brennstoff. Die Erfindung betrifft weiterhin eine Vorrichtung zur Durchführung des Verfahrens.

Kraftwerke, die mit Brennstoffen auf Kohlenstoffbasis betrieben werden, zählen zu den Hauptverursachern von Kohlenstoffdioxid.

Ein Verfahren und eine Vorrichtung der eingangs genannten Art sind aus der DE 10 2009 007 567 A1 bereits bekannt. Hier wird eine Verwertung von Kohlenstoffdioxid CO₂ aus Abgasen fossil befeuerter Kraftwerke und anderer Emittenten zur Herstellung von Methanol CH₃OH beschrieben. Dabei wird Strom, der durch regenerative Energiequellen erzeugt wird, zur Elektrolyse von Wasser eingesetzt. Der dabei gebildete Sauerstoff wird insbesondere zum Kraftwerk oder zum Emittenten geleitet und für einen Verbrennungsprozess genutzt. Der weiterhin erzeugte Wasserstoff wird mit aus dem Abgas des Verbrennungsprozesses stammendem Kohlenstoffdioxid gemäß folgender Reaktionsgleichung zu Methanol CH₃OH umgesetzt:

CO₂ + 3H₂ → CH₃OH + H₂O

Das Methanol wird optional zum Kraftwerk oder zum Emittenten geleitet und dem Verbrennungsprozess zugeführt.

Die FR 28 244 93 A1 beschreibt eine Verwertung von Kohlenstoffdioxid aus Industrieanlagen. Dabei wird zur Gewinnung von Wasserstoff und Sauerstoff eine Elektrolyse durchgeführt. Das in der Industrieanlage anfallende Kohlenstoffdioxid wird gemäß folgender Reaktionsgleichung mit dem Wasserstoff bei einer Temperatur im Bereich von 1000 bis 2000°C zu Wasser und (CO + 2H₂) bzw. Methanol umgesetzt:

CO₂ + 4H₂ + 0,5 O2 → 2H₂O + (CO + 2H₂)

Die WO 2007/108014 A1 offenbart ebenfalls eine Nutzung von aus industriellen Anlagen stammendem Kohlenstoffdioxid zur Herstellung von flüssigem Brennstoff, insbesondere Methanol. Der zur Umsetzung des Kohlenstoffdioxids benötigte Wasserstoff wird beispielsweise über eine bipolare alkalische Elektrolyseeinrichtung bereitgestellt.

Von Kraftwerken, die mit Brennstoffen auf Kohlenstoffbasis betrieben werden, werden neben stofflichen Emissionen in Form von Abgasen, Dämpfen, Feinstäuben, Abwässern und dergleichen auch Emissionen in Form von Energie, insbesondere Abwärme, Hörschall und dergleichen, an die Umwelt abgegeben. Die Abwärme kann dabei in einer stofflichen Emission gespeichert sein. Eine Abgabe der Abwärme an die Umgebung kann generell über Konvektion, Wärmeleitung oder Strahlung erfolgen.

So enthält das Abgas eines solchen Kraftwerks, neben dem bereits für eine Verwertung vorgesehenen Kohlenstoffdioxid, häufig beispielsweise große Mengen an Wasserdampf. Die Abwärme, zum Teil in Form von Verdampfungsenergie, sowie das Wasser und weitere Emissionen werden bisher nur unzureichend genutzt.

Es ist Aufgabe der Erfindung, ein Verfahren und eine Vorrichtung zur effizienteren Verwertung von zumindest einem Teil der Emissionen eines Kraftwerks bereitzustellen, das mit mindestens einem Brennstoff auf Kohlenstoffbasis betrieben wird.

Die Aufgabe wird für das Verfahren zur Verwertung von Emissionen eines mit mindestens einem Brennstoff auf Kohlenstoffbasis betriebenen Kraftwerks, umfassend mindestens einen Emittenten, welcher Emissionen umfassend ein Kohlenstoffdioxid enthaltendes Abgas erzeugt, mindestens eine Elektrolyseeinrichtung zur Zerlegung von Wasser in Wasserstoff und Sauerstoff, und mindestens einen, dem mindestens einen Emittenten und der mindestens einen Elektrolyseeinrichtung nachgeschalteten chemischen Reaktor zur Umsetzung zumindest eines Teils des Abgases und des Wasserstoffs in mindestens einen synthetischen Grund- oder Brennstoff, gelöst, indem der mindestens einen Elektrolyseeinrichtung und/oder dem mindestens einen chemischen Reaktor, zusätzlich zu einer ersten Emission umfassend mindestens den Teil des Abgases, mindestens eine zweite Emission des mindestens einen Emittenten zur Verwertung zugeführt wird.

Die Aufgabe wird für die Vorrichtung zur Durchführung des erfindungsgemäßen Verfahrens gelöst, indem sie folgendes umfasst:
- ein mit mindestens einem Brennstoff auf Kohlenstoffbasis betriebenes Kraftwerk, umfassend mindestens einen Emittenten, der Emissionen umfassend ein Kohlenstoffdioxid enthaltendes Abgas erzeugt,
- mindestens eine Elektrolyseeinrichtung zur Zerlegung von Wasser in Wasserstoff und Sauerstoff,
- mindestens einen, dem mindestens einen Emittenten und der mindestens einen Elektrolyseeinrichtung nachgeschalteten chemischen Reaktor,
- mindestens eine erste Einrichtung zur Zuführung einer ersten Emission umfassend mindestens einen Teil des Abgases zu der mindestens einen Elektrolyseeinrichtung und/oder dem mindestens einen chemischen Reaktor; und
- mindestens eine zweite Einrichtung zur Zuführung mindestens einer zweiten Emission des mindestens einen Emittenten zu der mindestens einen Elektrolyseeinrichtung und/oder dem mindestens einen chemischen Reaktor.

Ein Kraftwerk ist eine Anlage zur Stromerzeugung und zudem gegebenenfalls zur Bereitstellung von thermischer Leistung. Ein mit mindestens einem Brennstoff auf Kohlenstoffbasis betriebenes Kraftwerk ist beispielsweise ein Kohlekraftwerk, ein Gasturbinenkraftwerk oder ein Ölkraftwerk. Zur Befeuerung dieser werden fossile Brennstoffe wie Kohle, Erdöl oder Erdgas eingesetzt. Weiterhin sind auch Müllkraftwerke, Biomassekraftwerke, usw. Kraftwerke, die mit mindestens einem Brennstoff auf Kohlenstoffbasis betrieben werden.

Ein Emittent, welcher Emissionen umfassend ein Kohlenstoffdioxid enthaltendes Abgas erzeugt, ist in einem solchen Kraftwerk beispielsweise eine Brennkammer, die mit einem oder mehreren Brennern befeuert wird. Als Emittent wird hier auch eine Kombination einer Brennkammer mit mindestens einer der Brennkammer mittelbar oder unmittelbar nachgeordneten Turbine verstanden.

In mit Erdgas betriebenen Kraftwerken kann das Erdgas auch in einer Reformereinheit zu Wasserstoff und Kohlendioxid reformiert und lediglich der Wasserstoff verfeuert werden. Das Kohlendioxid kann abgetrennt und als Emission oder Bestandteil einer Emission verwertet werden, so dass auch eine solche Reformereinheit einen Emittenten bildet.

Als "Abgas" wird hierbei nicht nur eine Abgaskomponente oder eine Mischung diverser Abgaskomponenten verstanden, die unmittelbar aus einem Emittenten kommen, sondern auch deren Abbau- oder Folgeprodukte im Verfahrensablauf, bevor ein synthetischer Grund- oder Brennstoff hergestellt wurde. Beispielsweise kann die Abgaskomponente Kohlenstoffdioxid in einer Elektrolyseeinrichtung zumindest teilweise in Kohlenmonoxid umgewandelt werden. Das Kohlenmonoxid wird hierbei immer noch als "Abgas" angesehen.

Als Grund- oder Brennstoffe werden, sofern beispielsweise Kohlenstoffdioxid aus dem Abgas verwertet wird, Kohlenwasserstoffe und deren Derivate gebildet, die insbesondere in gasförmigem oder flüssigem Aggregatzustand vorliegen. Alkane, Alkohole usw. sind Beispiele für mittels des Verfahrens herstellbare Grund- oder Brennstoffe. Derartige Kohlenwasserstoffe weisen vorteilhafter Weise einen höheren spezifischen Energieinhalt auf als Wasserstoff. Wird Stickstoff aus dem Abgas verwertet, so kann Ammoniak als Grundstoff erzeugt werden.

Gemäß der Erfindung werden neben einer Verwertung einer ersten Emission umfassend mindestens einen Teil des Abgases weitere, eingangs beschriebene Emissionen des Kraftwerks verwertet. Dies erhöht die Effizienz des Kraftwerks, minimiert die Emissionsmengen zumindest bestimmter Emissionen und ermöglicht einen besonders kosten- und energiesparenden Kraftwerksbetrieb.

Bei einer bevorzugten Elektrolyseeinrichtung handelt es sich um eine Hochtemperatur-Elektrolyseeinheit mit einer Betriebstemperatur von ≥ 600°C, insbesondere im Bereich von 600 bis 1000°C. Besonders bevorzugt handelt es sich dabei um eine keramische Hochtemperatur-Elektrolyseeinheit, welche mit Wasser in dampfförmigem Zustand gespeist wird. Bei einer Dampfspeisung wird von einem hohen elektrischen Wirkungsgrad von bis zu 80 % ausgegangen. Eine keramische Hochtemperatur-Elektrolyseeinheit weist bevorzugt einen Sauerstoffionen leitenden Elektrolyten auf, insbesondere auf Basis von Yttrium-stabilisiertem Zirkondioxid. Der Betrieb einer Hochtemperatur-Elektrolyseeinheit erfolgt üblicherweise in einem Niederdruckbereich, insbesondere in einem Bereich von 1 bis 4 bar. Alternativ ist es aber ebenso möglich, eine Hochtemperatur-Elektrolyseeinheit in einem Druckbereich von bis zu etwa 100 bar zu betreiben.

Alternativ ist auch der Einsatz einer Elektrolyseeinrichtung in Form einer Niedertemperatur-Elektrolyseeinheit, insbesondere einer PEM-Elektrolyseeinheit (PEM = Proton Exchange Membrane) mit einer Betriebstemperatur im Bereich von 0 bis 130°C, insbesondere von 50 bis 90°C, von Vorteil. Der Elektrolyt besteht hier aus einer mechanisch verformbaren Polymermembrane und nicht, wie bei den oben genannten keramischen Elektrolyseeinheiten, aus einer vergleichsweise spröden Keramik. Eine PEM-Elektrolyseeinheit wird vorzugsweise in einem Druckbereich von 4 bis 100 bar, insbesondere von 10 bis 50 bar, mit Wasser in flüssigem Zustand betrieben. Das eingesetzte Wasser ist dabei frei von ionischen Verunreinigungen zu halten. Daher wird das Wasser in einer bevorzugten Ausgestaltung mittels mindestens einer Ionentauschereinheit gereinigt, bevor es in die Elektrolyseeinheit eingeleitet wird. Dabei werden spezifische elektrische Leitfähigkeiten des gereinigten Wassers im Bereich von < 1 µS/cm angestrebt.

Aufgrund der geringen Betriebstemperatur und der mechanischen Eigenschaften der verwendeten Komponenten sind Niedertemperatur-Elektrolyseeinrichtungen mit einem geringen technischen und betriebswirtschaftlichen Risiko betreibbar.

In einer bevorzugten Ausgestaltung des Verfahrens wird der mindestens einen Elektrolyseeinrichtung und/oder dem mindestens einen chemischen Reaktor, zusätzlich zu einer diesen optional über die erste Emission zugeführten ersten Wärmemenge, eine zumindest zum Teil aus einer zweiten Emission in Form von Abwärme des mindestens einen Emittenten gewonnene zweite Wärmemenge zugeführt.

So kann der verwertete Teil des Abgases im Hinblick auf ein Temperaturniveau unmittelbar beim Austritt des Abgases aus dem Emittenten in abgekühltem Zustand oder in vergleichbar heißem Zustand, also inklusive einer beträchtlichen ersten Wärmemenge, aus dem Emittenten in die mindestens eine Elektrolyseeinrichtung und/oder den mindestens einen chemischen Reaktor eingespeist werden.

Eine Abkühlung gegenüber dem Temperaturniveau unmittelbar beim Austritt des Abgases aus dem Emittenten kann beispielsweise durch eine Abgasreinigung, Abgasseparation, beispielsweise zur Abtrennung von Kohlenstoffdioxid, Stickstoff usw. auftreten. Insbesondere wird bisher beispielsweise Wasserdampf, der sich, z.B. während eines Verbrennungsprozesses, in einem Emittenten bildet, abgetrennt und abgeführt. Die in dieser stofflichen zweiten Emission enthaltene zweite Wärmemenge wird nun vorzugsweise im Bereich einer Elektrolyseeinrichtung und/oder eines chemischen Reaktors verwertet.

Weiterhin wird insbesondere auch Abwärme, die über Wärmeübertragungseinheiten, wie z.B. Kühleinheiten oder Wärmetauschereinheiten, von einem Emittenten abgeführt oder unmittelbar an die Umgebung abgegeben wird, nun als zweite Emission im weiteren Verfahrensablauf im Bereich der mindestens einen Elektrolyseeinrichtung und/oder des mindestens einen chemischen Reaktors verwertet.

Es hat sich bewährt, wenn eine zweite Emission in Form von Abwärme zumindest eines Bauteils des mindestens einen Emittenten zur Beheizung der mindestens einen Elektrolyseeinrichtung und/oder des mindestens einen chemischen Reaktors eingesetzt wird.

So kann beispielsweise Abwärme, welche über häufig zur Kühlung von Brennern vorgesehene Kühleinrichtungen vom Emittenten abgeführt wird, der mindestens einen Elektrolyseeinrichtung und/oder dem mindestens einen chemischen Reaktor zugeführt werden.

Vorzugsweise wird die mindestens eine Elektrolyseeinrichtung zumindest teilweise mit einer zweiten Emission in Form von Wasser betrieben, das aus dem mindestens einen Emittenten stammt. Dabei ist es von Vorteil, wenn mindestens eine Zuführleitung für das Wasser aus dem Emittenten zu der mindestens einen Elektrolyseeinrichtung vorhanden ist.

In einer besonders bevorzugten Ausgestaltung der Erfindung wird das in der ersten Emission enthaltene Kohlenstoffdioxid direkt an einer negativen Elektrode einer Hochtemperatur-Elektrolyseeinrichtung zusammen mit Wasser in dampfförmigem Zustand, das aus dem Emittenten stammt, umgesetzt.

Eine zweite Emission in Form von Abwärme des mindestens einen Emittenten kann auch zumindest teilweise dazu eingesetzt werden, außerhalb des mindestens einen Emittenten Wasserdampf zu erzeugen, mit welchem eine Elektrolyseeinrichtung, insbesondere Hochtemperatur-Elektrolyseeinrichtung, zumindest teilweise gespeist wird.

Auch ein Einsatz von erwärmtem Brauchwasser des Kraftwerks, insbesondere aus Kühleinrichtungen des Emittenten, als Wasser zur Speisung einer Elektrolyseeinrichtung ist möglich. Je nach verwendeter Elektrolyseeinrichtung ist dabei gegebenenfalls eine Reinigung des Wassers, beispielsweise über Filter und/oder Ionentauschereinheiten, erforderlich.

Diese Maßnamen ermöglichen Einsparungen beim Einsatz von Frisch- bzw. Trinkwasser.

In einer besonders bevorzugten weiteren Ausgestaltung der Erfindung wird eine erste Emission umfassend Kohlenstoffdioxid und eine zweite Emission in Form von Abwärme direkt einem chemischen Reaktor zugeführt und dort mit Wasserstoff, der aus einer Niedertemperatur-Elektrolyseeinheit stammt, umgesetzt.

Wird eine zweite Emission in Form von Abwärme zumindest teilweise dem mindestens einen chemischen Reaktor zu dessen Beheizung zugeführt, so entfällt ein zusätzlicher Energiebedarf und die Kosten für den Reaktorbetrieb können gesenkt werden.

Der in der mindestens einen Elektrolyseeinrichtung erzeugte Sauerstoff wird in einer besonders bevorzugten Ausgestaltung der Erfindung dem mindestens einen Emittenten zumindest teilweise als Oxidationsmittel, z.B. zu dessen Befeuerung, zugeführt. In einer bevorzugten Ausgestaltung der Vorrichtung ist diese dazu mit mindestens einer Gasleitung ausgestattet, über welche der Sauerstoff von der Elektrolyseeinrichtung zum Emittenten geleitet wird. Der Sauerstoff kann dabei in einem Speichertank zwischengespeichert werden. Eine Befeuerung von Emittenten mit reinem Sauerstoff oder einer Mischung aus Luft und Sauerstoff ist besonders bevorzugt, da dies zu einem hohen Anteil an effizient verwertbarem reinem Kohlenstoffdioxid im Abgas führt.

Weiterhin hat es sich bewährt, wenn der mindestens eine synthetische Grund- oder Brennstoff zumindest teilweise zur Befeuerung des mindestens einen Emittenten eingesetzt wird. Der Kohlenstoff aus dem ursprünglich verfeuerten Brennstoff wird dadurch im Kreis geführt und eine Umweltbelastung durch hohe Mengen an Kohlenstoffdioxid verhindert.

Neben einer Verwertung von Emissionen in Form von Kohlenstoffdioxid, Wasser und Abwärme können weitere Emissionen aus Verbrennungsvorgängen, wie beispielsweise Stickstoff usw. mit elektrolytisch erzeugtem Wasserstoff in einen synthetischen Grund- oder Brennstoff umgesetzt werden. Stickstoff, welcher z.B. aus eingesetzter Verbrennungsluft stammt, kann beispielsweise in Ammoniak NH₃ umgesetzt. Dabei werden jeweils auf den zu erzeugenden synthetischen Grund- oder Brennstoff abgestimmte chemische Reaktoren eingesetzt.

Dabei hat es sich als vorteilhaft erwiesen, wenn der mindestens einen Elektrolyseeinrichtung mindestens zwei unterschiedliche chemische Reaktoren zur Umsetzung von zumindest zwei gleichen oder unterschiedlichen Teilen des Abgases und des Wasserstoffs in mindestens zwei unterschiedliche synthetische Grund- oder Brennstoffe nachgeordnet betrieben werden. Besonders bevorzugt wird als erster Teil des Abgases Kohlenstoffdioxid in einem ersten chemischen Reaktor mit elektrolytisch erzeugtem Wasserstoff in eine Kohlenwasserstoffverbindung oder ein Kohlenwasserstoffderivat umgesetzt wird und als zweiter Teil des Abgases Stickstoff in einem zweiten Reaktor mit elektrolytisch erzeugtem Wasserstoff zu Ammoniak umgesetzt. Aber auch eine Umsetzung von in zwei Stoffströme aufgeteiltem Kohlenstoffdioxid aus dem Abgas in zwei unterschiedlichen chemischen Reaktoren zu unterschiedlichen Kohlenwasserstoffverbindungen oder Kohlenwasserstoffderivaten ist möglich.

Die mindestens zwei chemischen Reaktoren weisen bevorzugt unterschiedliche Katalysatormaterialien auf und sind zur Umsetzung gleicher oder unterschiedlicher Teile des Abgases in unterschiedliche synthetische Grund- oder Brennstoffe eingerichtet.

Weiterhin ist es von Vorteil, wenn die Vorrichtung mit einer Umschalteinrichtung ausgestattet wird, mit welcher eine Auswahl eines bestimmten chemischen Reaktors aus einer Anzahl an zur Verfügung stehenden unterschiedlichen chemischen Reaktoren bzw. ein Abschalten eines bestimmten chemischen Reaktors möglich ist. Dies kann gewünscht sein, wenn ein bestimmter Grund- oder Brennstoff gerade besonders dringend bzw. nicht benötigt wird.

Es ist besonders bevorzugt, die mindestens eine Elektrolyseeinrichtung mit elektrischer Energie aus einer regenerativen Energiequelle zu versorgen, da diese Energiequellen bei der Stromproduktion im Gegensatz zu einem konventionellen, fossil befeuerten Kraftwerken nachhaltig sind und kein Kohlenstoffdioxid freisetzen.

Der Bau von Anlagen zur Gewinnung regenerativer Energien, wie geothermaler Energie, Wasserkraft, insbesondere von Windenergie, Sonnenenergie, usw. hat in den letzten Jahren stark zugenommen. Allerdings ist die naturgegebene Angebotssituation, insbesondere im Hinblick auf die Windenergie, für derartige Anlagen oft schwer prognostizierbar. Dadurch treten witterungsbedingt, beispielsweise in Starkwind - bzw. Windflautezeiten, Energieerzeugungsspitzen bzw. Energieerzeugungstäler auf.

Zur Überbrückung von Stark- und Schwachlastphasen ist es daher von Vorteil, wenn z.B. Kohlenstoffdioxid aus dem Emittenten sowie elektrolytisch gewonnener Sauerstoff und Wasserstoff in entsprechenden Speicheranlagen auf Vorrat hinterlegt bzw. vorgehalten werden.

Vorzugsweise werden die mindestens eine Elektrolyseeinrichtung und der mindestens eine chemische Reaktor auf gleichem oder ähnlichem Druckniveau betrieben. So werden chemische Reaktoren üblicherweise unter Druck betrieben. Das Druckniveau der Elektrolyseeinrichtung wird diesem bevorzugt angepasst oder in etwa angepasst. Aufgrund einer dadurch erzielbaren höheren Speicherdichte können die Betriebskosten gesenkt werden.

Alternativ ist es jedoch ebenso möglich, wenn die mindestens eine Elektrolyseeinrichtung und der mindestens eine chemische Reaktor auf unterschiedlichen Druckniveaus betrieben werden. In diesem Fall wird zwischen einer Elektrolyseeinrichtung und einem chemischen Reaktor mindestens ein Kompressor oder mindestens eine Druckreduziereinrichtung zwischengeschaltet, um den Übergang zwischen den unterschiedlichen Druckniveaus zu realisieren.

Die mindestens eine zweite Einrichtung, über welche der mindestens einen Elektrolyseeinrichtung und/oder dem mindestens einen chemischen Reaktor die mindestens eine zweite Emission zugeführt wird, umfasst bevorzugt mindestens eine Wärmeübertragungseinheit, welche eingerichtet ist, eine zweite Emission in Form von Abwärme zumindest eines Bauteils des Emittenten zumindest teilweise an die mindestens eine Elektrolyseeinrichtung und/oder den mindestens einen chemischen Reaktor zu übertragen. Es kann sich bei der Wärmeübertragungseinheit um einen Wärmetauscher, ein Gebläse, eine Kondensiereinheit, einen Wasserdampferzeuger usw. handeln.

Insbesondere umfasst die mindestens eine zweite Einrichtung mindestens eine Wärmeübertragungseinheit in Form eines Wasserdampferzeugers, welcher eingerichtet ist, mittels einer zweiten Emission in Form von Abwärme des mindestens einen Emittenten außerhalb des mindestens einen Emittenten Wasserdampf zu erzeugen und diesen zumindest teilweise der mindestens einen Elektrolyseeinrichtung zu deren Speisung zuzuführen. Dies ist insbesondere bei Verwendung von Hochtemperatur-Elektrolyseeinheiten, welche mit Wasser in dampfförmigem Zustand gespeist werden, von Interesse.

Es hat sich bewährt, wenn mindestens eine Sammeleinrichtung zur Speicherung des erzeugten mindestens einen synthetischen Grund- oder Brennstoffs vorhanden ist. Der mindestens eine synthetische Grund- oder Brennstoff kann, gegebenenfalls nach einer weiteren Aufbereitung, in Transportbehälter abgefüllt werden, um ihn an andere Orte zu transportieren. Hierzu ist bei flüssigen synthetischen Grund- oder Brennstoffen eine weniger komplizierte und lediglich ein geringeres Gefahrenpotential aufweisende Transporttechnologie anwendbar, wie sie beispielsweise bei einem Transport von Wasserstoff notwendig wäre. Reiner Wasserstoff ist bekanntermaßen hochentzündlich, hochexplosiv und zudem schwierig in der Lagerung.

Die mindestens eine Sammeleinrichtung ist bevorzugt mittels mindestens einer Versorgungsleitung mit dem mindestens einen Emittenten verbunden, über welche der mindestens eine synthetische Grund- oder Brennstoff - falls dafür einsetzbar - der Befeuerung des mindestens einen Emittenten zuführbar ist.

Die Figuren 1 bis 5 sollen die Erfindung beispielhaft erläutern. So zeigt
- FIG 1: ein erstes Verfahren und eine erste Vorrichtung zur Verwertung von Emissionen eines Kraftwerks;
- FIG 2: ein zweites Verfahren und eine zweite Vorrichtung zur Verwertung von Emissionen eines Kraftwerks;
- FIG 3: ein drittes Verfahren und eine dritte Vorrichtung zur Verwertung von Emissionen eines Kraftwerks;
- FIG 4: ein viertes Verfahren und eine vierte Vorrichtung zur Verwertung von Emissionen eines Kraftwerks; und
- FIG 5: ein fünftes Verfahren und eine fünfte Vorrichtung zur Verwertung von Emissionen eines Kraftwerks.

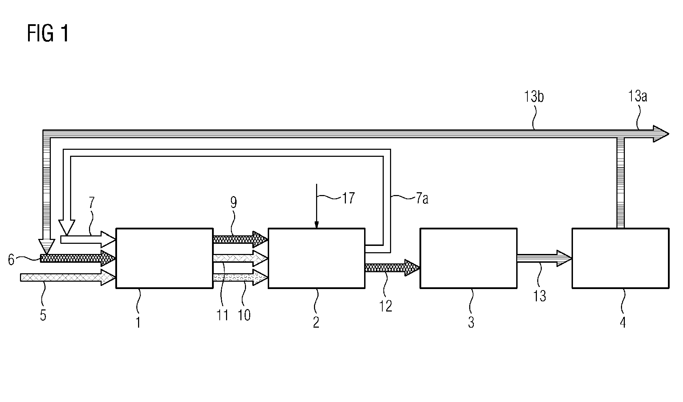

FIG 1 zeigt ein erstes Verfahren und eine erste Vorrichtung zur Verwertung von Emissionen eines Kraftwerks, das mit einem Brennstoff 6 auf Kohlenstoffbasis betrieben wird. Das hier nicht näher dargestellte Kraftwerk umfasst einen Emittenten 1, der mit dem Brennstoff 6 befeuert wird und ein Kohlenstoffdioxid enthaltendes Abgas erzeugt. Bei dem Emittenten 1 handelt es sich um eine Kombination einer Brennkammer, die mit mindestens einem Brenner oder mehreren Brennern befeuert ist, mit einer der Brennkammer nachgeschalteten Turbine. Als Brennstoff 6 auf Kohlenstoffbasis wird hier Kohle, Erdgas, Erdöl, Abfall, Biomasse und dergleichen verfeuert. Als Oxidationsmittel 7 werden zur Verfeuerung des Brennstoffs 6 in der Regel Luft oder bevorzugt auch Sauerstoff oder LuftSauerstoff-Mischungen eingesetzt.

Dem Emittenten 1 ist eine Elektrolyseeinrichtung 2 zur Zerlegung von Wasser in Wasserstoff und Sauerstoff 7a nachgeschaltet. Die Elektrolyseeinrichtung 2 wird dabei durch eine regenerative Energiequelle mit Energie 17 versorgt. Bei der Elektrolyseeinrichtung 2 handelt es sich hier um eine keramische Hochtemperatur-Elektrolyseeinheit, welcher eine erste Emission des Emittenten 1 umfassend Kohlenstoffdioxid 9 zugeführt wird. Weiterhin wird der Elektrolyseeinrichtung 2 eine zweite Emission aus dem Abgas des Emittenten 1 zugeführt, und zwar Wasser in dampfförmigem Zustand 10, das unmittelbar elektrolytisch zerlegt wird. Eine weitere zweite Emission des Emittenten 1 in Form von Abwärme 11 wird der Elektrolyseeinrichtung 2 zu deren Beheizung zugeführt.

Der von der Elektrolyseeinrichtung 2 erzeugte Sauerstoff 7a wird optional als Oxidationsmittel für die Verbrennung des Brennstoffs 6 eingesetzt.

Das Gasgemisch 12 umfassend Wasserstoff und Kohlenstoffdioxid wird einem chemischen Reaktor 3 zugeführt, wo eine Umsetzung in einen synthetischen Grund- oder Brennstoff 13, beispielsweise in Form von Methan oder Methanol, erfolgt.

Der, der Elektrolyseeinrichtung 2 nachgeschaltete chemische Reaktor 3 weist, je nach umzusetzender Emission und gewünschtem synthetischen Grund- oder Brennstoff, geeignete Katalysatoren, wie z.B. Metalloxide in Form von NiO oder CuO, auf und wird hier auf gleichem Druckniveau betrieben wie die Elektrolyseeinrichtung 2.

Zur Zuführung der ersten Emission umfassend das Kohlenstoffdioxid 9 zu der mindestens einen Elektrolyseeinrichtung 2 ist mindestens eine erste Einrichtung, beispielsweise in Form eines Rohres, gegebenenfalls umfassend diverse Filter- und/oder Trenneinheiten, vorhanden. Eine derartige Filter- und/oder Trenneinheit kann beispielsweise zur Abtrennung von Feinstaub, Wasserdampf usw. dienen.

Zur Zuführung von zweiten Emissionen in Form von Abwärme 11 und Wasser in dampfförmigem Zustand 10 zur Elektrolyseeinrichtung 2 ist mindestens eine zweite Einrichtung, beispielsweise in Form von Rohren, Wärmetauschern usw. vorhanden.

Der erzeugte mindestens eine Grund- oder Brennstoff 13 wird in einer Sammeleinrichtung 4 aufgefangen und entweder außerhalb des Prozesses verwendet, 13a, und/oder - zumindest teilweise - dem Emittenten 1 zusätzlich zum Brennstoff 6 als synthetischer Brennstoff 13b wieder zugeführt.

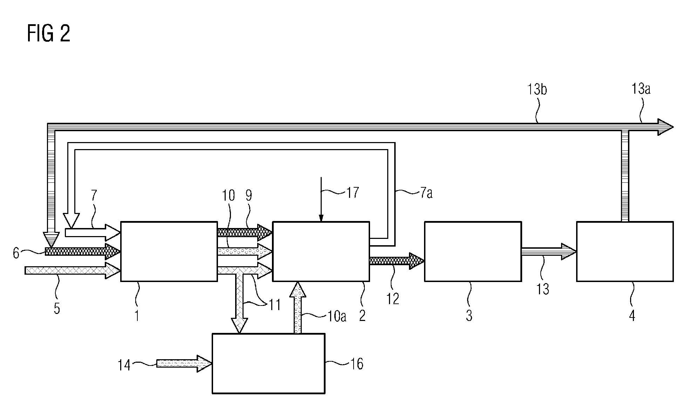

FIG 2 zeigt ein zweites Verfahren und eine zweite Vorrichtung zur Verwertung von Emissionen eines Kraftwerks. Gleiche Bezugszeichen wie in FIG 1 bezeichnen gleiche Elemente. In dieser Ausführungsform wird im Unterschied zu der in FIG 1 gezeigten Ausführungsform ein Teil der Abwärme 11 des Emittenten 1 dazu verwendet, Wasser in flüssigem Zustand 14 in einer Wärmeübertragungseinheit in Form eines Wasserdampferzeugers 16 zu verdampfen und anschließend der Elektrolyseeinrichtung 2 zuzuführen. Diese Vorgehensweise bietet sich insbesondere dann an, wenn nicht die in der Elektrolyseeinrichtung 2 benötigte Menge an Wasserdampf 10 aus dem Emittenten 1 angeliefert werden kann und/oder die Abwärme 11 im Überschuss zur Verfügung steht und nicht gänzlich zur Beheizung der Hochtemperatur-Elektrolyseeinheit gebraucht wird.

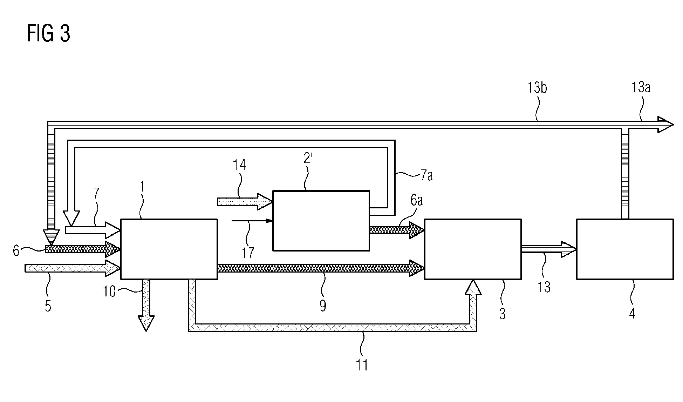

FIG 3 zeigt ein drittes Verfahren und eine dritte Vorrichtung zur Verwertung von Emissionen eines Kraftwerks. Gleiche Bezugszeichen wie in FIG 1 bezeichnen gleiche Elemente. In dieser Ausführungsform wird im Unterschied zu der in FIG 1 gezeigten Ausführungsform eine Elektrolyseeinrichtung 2' in Form einer Niedertemperatur- Elektrolyseeinheit, im Detail eine PEM-Elektrolyseeinheit, zur Erzeugung von Wasserstoff 6a und Sauerstoff 7a eingesetzt. Diese wird bei einer Betriebstemperatur im Bereich von 0 bis 130°C, insbesondere von 50 bis 90°C, und in einem Druckbereich von 4 bis 100 bar, insbesondere von 10 bis 50 bar, mit Wasser (in flüssigem Zustand) 14 betrieben. Der von der Elektrolyseeinrichtung 2' erzeugte Sauerstoff 7a wird optional als Oxidationsmittel 7 für die Verbrennung des Brennstoffs 6 eingesetzt.

Der elektrolytisch erzeugte Wasserstoff 6a und das Kohlenstoffdioxid 9 werden getrennt einem chemischen Reaktor 3 zugeführt, wo eine Umsetzung in einen synthetischen Grund- oder Brennstoff 13 erfolgt.

Der, der Elektrolyseeinrichtung 2' nachgeschaltete chemische Reaktor 3 weist einen geeigneten Katalysator auf und wird auf gleichem Druckniveau betrieben wie die Elektrolyseeinrichtung 2'.

Zumindest ein Teil der Abwärme 11 des Emittenten 1 wird unabhängig von der ersten Emission enthaltend das Kohlenstoffdioxid 9 unmittelbar dem chemischen Reaktor 3 zu dessen Beheizung zugeführt.

Der erzeugte Grund- oder Brennstoff 13 wird in einer Sammeleinrichtung 4 aufgefangen und außerhalb des Prozesses verwendet, 13a, oder dem Emittenten 1 zusätzlich zum Brennstoff 6 als synthetischer Brennstoff 13b zugeführt.

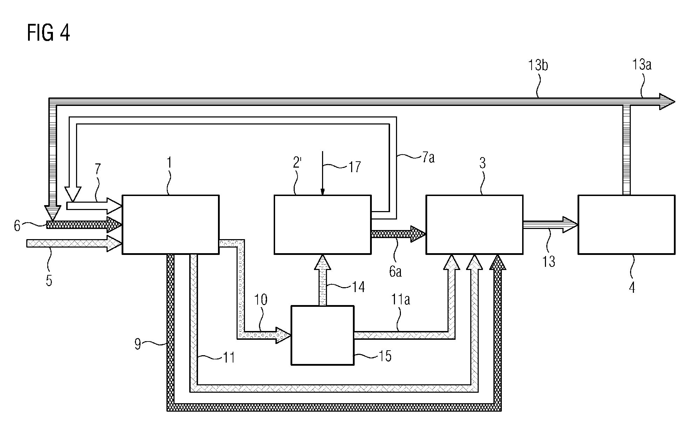

FIG 4 zeigt ein viertes Verfahren und eine vierte Vorrichtung zur Verwertung von Emissionen eines Kraftwerks. Gleiche Bezugszeichen wie in FIG 3 bezeichnen gleiche Elemente. In dieser Ausführungsform wird im Unterschied zu der in FIG 3 gezeigten Ausführungsform Wasser in dampfförmigem Zustand 10 aus dem Emittenten 1 einer Kondensiereinheit 15 zugeführt und das gewonnene Wasser in flüssigem Zustand 14 der Elektrolyseeinrichtung 2' zugeführt. Dieses Wasser ist sauber und unmittelbar verwendbar, so dass eine Reinigung mittels Ionentauschereinheiten entfallen kann.

Zumindest ein Teil der Abwärme 11 des Emittenten 1 sowie die über die Kondensiereinheit 15 dem Wasser in dampfförmigem Zustand 10 entzogene Abwärme 11a wird unabhängig von der ersten Emission umfassend das Kohlenstoffdioxid 9 unmittelbar dem chemischen Reaktor 3 zu dessen Beheizung zugeführt.

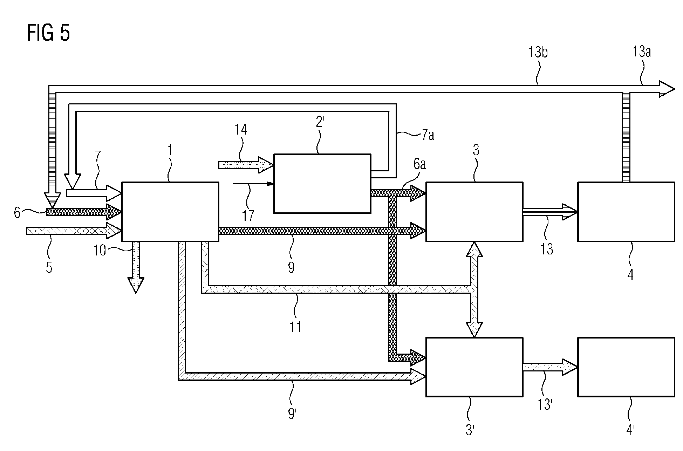

FIG 5 beschreibt ein fünftes Verfahren und eine fünfte Vorrichtung zur Verwertung von Emissionen eines Kraftwerks. Gleiche Bezugszeichen wie in FIG 3 bezeichnen gleiche Elemente. In dieser Ausführungsform wird im Unterschied zu der in FIG 3 gezeigten Ausführungsform ein zweiter chemischer Reaktor 3' vorgesehen, welcher dem Emittenten 1 und der Elektrolyseeinrichtung 2' zusätzlich nachgeschaltet ist. Dem ersten chemischen Reaktor 3 wird eine erste Emission umfassend Kohlenstoffdioxid 9 aus dem Abgas des Emittenten 1 zugeführt, das mit Wasserstoff 6a aus der Elektrolyseeinrichtung 2' zu einem ersten Grund- oder Brennstoff 13, beispielsweise Methan oder Methanol, umgesetzt wird. Dem zweiten chemischen Reaktor 3' wird eine weitere erste Emission umfassend Stickstoff 9' aus dem Abgas des Emittenten 1 zugeführt, das mit Wasserstoff 6a aus der Elektrolyseeinrichtung 2' zu einem zweiten Grund-oder Brennstoff 13' in Form von Ammoniak umgesetzt wird.

Als zweite Emission wird zumindest ein Teil der Abwärme 11 des Emittenten 1 unmittelbar den chemischen Reaktoren 3, 3' zu deren Beheizung zugeführt.

Der erzeugte erste Grund- oder Brennstoff 13 wird in einer ersten Sammeleinrichtung 4 aufgefangen und außerhalb des Prozesses verwendet, 13a, oder dem Emittenten 1 zusätzlich zum Brennstoff 6 als synthetischer Brennstoff 13b zugeführt. Der erzeugte zweite Grund- oder Brennstoff 13' wird in einer zweiten Sammeleinrichtung 4' aufgefangen und außerhalb des Prozesses verwendet.

Die in den Figuren 1 bis 5 dargestellten Verfahren und Vorrichtungen können von einem Fachmann jederzeit modifiziert werden, ohne den Gedanken der Erfindung zu verlassen. So kann das Abgas eines oder mehrerer Emittenten parallel mehreren Elektrolyseeinrichtungen und/oder chemischen Reaktoren zugeführt werden, das Abgas mehrerer Emittenten lediglich einer Elektrolyseeinrichtung und/oder einem chemischen Reaktor zugeführt werden, die Anzahl an Elektrolyseeinrichtungen und chemischen Reaktoren unterschiedlich gewählt sein usw.

Die konkret erforderliche Ausgestaltung einer Vorrichtung hängt wesentlich von den zu verarbeitenden Emissionsmengen, der Leistung der eingesetzten Elektrolyseeinrichtung und der Leistung des jeweiligen chemischen Reaktors ab. Es können weiterhin auch andere als die genannten Typen von Elektrolyseeinrichtungen oder gleichzeitig unterschiedliche Typen von Elektrolyseeinrichtungen eingesetzt werden. Die verwendeten chemischen Reaktoren können sich in Aufbau und eingesetztem Katalysatormaterial unterscheiden, da diese je nach damit zu behandelnder Emission und gewünschtem Grund- oder Brennstoff auszuwählen sind.

## Patentansprüche

1. Verfahren zur Verwertung von Emissionen eines mit mindestens einem Brennstoff auf Kohlenstoffbasis betriebenen Kraftwerks, umfassend mindestens einen Emittenten (1), welcher Emissionen umfassend ein Kohlenstoffdioxid enthaltendes Abgas erzeugt, mindestens eine Elektrolyseeinrichtung (2, 2') zur Zerlegung von Wasser in Wasserstoff (6a) und Sauerstoff (7a), und mindestens einen, dem mindestens einen Emittenten (1) und der mindestens einen Elektrolyseeinrichtung (2, 2') nachgeschalteten chemischen Reaktor (3, 3') zur Umsetzung zumindest eines Teils des Abgases und des Wasserstoffs (6a) in mindestens einen synthetischen Grund- oder Brennstoff (13, 13'), **dadurch gekennzeichnet, dass** der mindestens einen Elektrolyseeinrichtung (2, 2') und/oder dem mindestens einen chemischen Reaktor (3, 3'), zusätzlich zu einer ersten Emission umfassend mindestens den Teil des Abgases, mindestens eine zweite Emission des mindestens einen Emittenten (1) zur Verwertung zugeführt wird.

2. Verfahren nach Anspruch 1,
**dadurch gekennzeichnet, dass** der mindestens einen Elektrolyseeinrichtung (2) und/oder dem mindestens einen chemischen Reaktor (3), zusätzlich von einer diesen optional über die erste Emission zugeführten ersten Wärmemenge, eine zumindest zum Teil aus der zweiten Emission in Form von Abwärme (11) des mindestens einen Emittenten (1) gewonnene zweite Wärmemenge zugeführt wird.

3. Verfahren nach Anspruch 1 oder Anspruch 2,
**dadurch gekennzeichnet, dass** die zweite Emission in Form von Abwärme (11) zumindest eines Bauteils des mindestens einen Emittenten (1) zur Beheizung der mindestens einen Elektrolyseeinrichtung (2) und/oder des mindestens einen chemischen Reaktors (3) eingesetzt wird.

4. Verfahren nach einem der Ansprüche 1 bis 3,
**dadurch gekennzeichnet, dass** die mindestens eine Elektrolyseeinrichtung (2, 2') zumindest teilweise mit der zweiten Emission in Form von Wasser betrieben wird, das aus dem mindestens einen Emittenten (1) stammt.

5. Verfahren nach einem der Ansprüche 1 bis 4,
**dadurch gekennzeichnet, dass** eine zweite Emission in Form von Abwärme (11) des mindestens einen Emittenten (1) zumindest teilweise dazu eingesetzt wird, außerhalb des Emittenten (1) Wasserdampf (10a) zu erzeugen, mit welchem die mindestens eine Elektrolyseeinrichtung (2) gespeist wird.

6. Verfahren nach einem der Ansprüche 1 bis 5,
**dadurch gekennzeichnet, dass** der in der mindestens einen Elektrolyseeinrichtung (2, 2') erzeugte Sauerstoff (7a) dem mindestens einen Emittenten (1) zumindest teilweise als Oxidationsmittel (7) zu dessen Befeuerung zugeführt wird.

7. Verfahren nach einem der Ansprüche 1 bis 6,
**dadurch gekennzeichnet, dass** die mindestens eine Elektrolyseeinrichtung (2, 2') mit elektrischer Energie (17) aus einer regenerativen Energiequelle versorgt wird.

8. Verfahren nach einem der Ansprüche 1 bis 7,
**dadurch gekennzeichnet, dass** die erste Emission als Teil des Abgases Kohlenstoffdioxid (9) umfasst und dass der damit gebildete synthetische Grund- oder Brennstoff (13) teilweise zur Befeuerung des mindestens einen Emittenten (1) eingesetzt wird.

9. Verfahren nach einem der Ansprüche 1 bis 8,
**dadurch gekennzeichnet, dass** der mindestens einen Elektrolyseeinrichtung (2') mindestens zwei unterschiedliche chemische Reaktoren (3, 3') zur Umsetzung von zumindest zwei gleichen oder unterschiedlichen Teilen des Abgases und des Wasserstoffs (6a) in mindestens zwei unterschiedliche synthetische Grund- oder Brennstoff (13, 13') nachgeordnet betrieben werden.

10. Verfahren nach Anspruch 9,
**dadurch gekennzeichnet, dass** als erster Teil des Abgases Kohlenstoffdioxid in einem ersten chemischen Reaktor (3) mit Wasserstoff (6a) in eine Kohlenwasserstoffverbindung oder ein Kohlenwasserstoffderivat umgesetzt wird und als zweiter Teil des Abgases Stickstoff in einem zweiten chemischen Reaktor (3') mit Wasserstoff (6a) zu Ammoniak umgesetzt wird.

11. Verfahren nach einem der Ansprüche 1 bis 10,
**dadurch gekennzeichnet, dass** die mindestens eine Elektrolyseeinrichtung (2) und der mindestens eine chemische Reaktor (3, 3') auf gleichem Druckniveau betrieben werden.

12. Vorrichtung zur Durchführung eines Verfahrens nach einem der Ansprüche 1 bis 11, umfassend:
- ein mit mindestens einem Brennstoff auf Kohlenstoffbasis betriebenes Kraftwerk, umfassend mindestens einen Emittenten (1), der Emissionen umfassend ein Kohlenstoffdioxid (9) enthaltendes Abgas erzeugt,
- mindestens eine Elektrolyseeinrichtung (2, 2') zur Zerlegung von Wasser in Wasserstoff (6a) und Sauerstoff (7a),
- mindestens einen, dem mindestens einen Emittenten (1) und der mindestens einen Elektrolyseeinrichtung (2, 2') nachgeschalteten chemischen Reaktor (3, 3'),
- mindestens eine erste Einrichtung zur Zuführung einer ersten Emission umfassend mindestens einen Teil des Abgases zu der mindestens einen Elektrolyseeinrichtung (2, 2') und/oder dem mindestens einen chemischen Reaktor (3, 3'); und
- mindestens eine zweite Einrichtung zur Zuführung mindestens einer zweiten Emission des mindestens einen Emittenten (1) zu der mindestens einen Elektrolyseeinrichtung (2, 2') und/oder dem mindestens einen chemischen Reaktor (3, 3').

13. Vorrichtung nach Anspruch 12,
wobei es sich bei dem mindestens einen Emittenten (1) um eine Brennkammer oder um eine Brennkammer mit mindestens einer der Brennkammer nachgeschalteten Turbine oder um eine Reformereinheit handelt.

14. Vorrichtung nach Anspruch 12 oder Anspruch 13,
wobei die mindestens eine Elektrolyseeinrichtung (2) durch eine Hochtemperatur-Elektrolyseeinheit oder durch eine Niedertemperatur-Elektrolyseeinheit, insbesondere eine PEM-Elektrolyseeinheit, gebildet ist.

15. Vorrichtung nach Anspruch 12 oder Anspruch 14,
wobei die mindestens eine zweite Einrichtung mindestens eine Wärmeübertragungseinheit umfasst, welche eingerichtet ist, eine zweite Emission in Form von Abwärme (11, 11a) des mindestens einen Emittenten (1) zumindest teilweise an die mindestens eine Elektrolyseeinrichtung (2) oder den mindestens einen chemischen Reaktor (3) zu übertragen.

16. Vorrichtung nach einem der Ansprüche 12 bis 15,
wobei mindestens eine Zuführleitung für eine zweite Emission in Form von Wasser zu der mindestens einen Elektrolyseeinrichtung (2) vorhanden ist, das zumindest teilweise aus einem Emittenten (1) stammt.

17. Vorrichtung nach einem der Ansprüche 12 bis 16,
wobei die mindestens eine zweite Einrichtung mindestens einen Wasserdampferzeuger (16) umfasst, welcher eingerichtet ist, mittels einer zweiten Emission in Form von Abwärme (11) des mindestens einen Emittenten (1) außerhalb des Emittenten (1) Wasserdampf (10a) zu erzeugen und diesen zumindest teilweise der mindestens einen Elektrolyseeinrichtung (2) zu deren Speisung zuzuführen.

18. Vorrichtung nach einem der Ansprüche 12 bis 17,
wobei mindestens eine Gasleitung vorhanden ist, über welche der in der mindestens einen Elektrolyseeinrichtung (2, 2') erzeugte Sauerstoff (7a) dem mindestens einen Emittenten (1) zumindest teilweise als Oxidationsmittel (7) zu dessen Befeuerung zuführbar ist.

19. Vorrichtung nach einem der Ansprüche 12 bis 18,
wobei mindestens zwei chemische Reaktoren (3, 3') vorhanden sind, die unterschiedliche Katalysatormaterialien aufweisen und zur Umsetzung gleicher oder unterschiedlicher Teile des Abgases in unterschiedliche synthetische Grund- oder Brennstoffe (13, 13') eingerichtet sind.

20. Vorrichtung nach einem der Ansprüche 12 bis 19,
wobei mindestens eine Sammeleinrichtung (4, 4') zur Speicherung des mindestens einen synthetischen Grund- oder Brennstoffs (13, 13') vorhanden ist.

21. Vorrichtung nach Anspruch 20,
wobei die mindestens eine Sammeleinrichtung (4) über mindestens eine Versorgungsleitung mit dem mindestens einen Emittenten (1) verbunden ist, über welche ein synthetischer Grund- oder Brennstoff (13) der Befeuerung des mindestens einen Emittenten (1) zuführbar ist.
